# EUROPÄISCHE PATENTSCHRIFT

(11) **EP 1 280 754 B1**
(45) Veröffentlichungstag und Bekanntmachung des Hinweises auf die Patenterteilung: **04.08.2004**
(21) Anmeldenummer: 01940459.9
(22) Anmeldetag: 11.05.2001
(51) Int. Cl.: C07C 45/50, B01J 31/24, C07F 15/00

(54) **METALL-KOMPLEX, DER EINEN 2-PHOSPHA-TRICYCLO[3.3.1.1 3,7 ]DECYLREST ALS LIGANDEN TRÄGT IN DER HYDROFORMYLIERUNG**
METAL COMPLEX CARRYING A 2-PHOSPHA-TRICYCLO 3.3.1.1 (3,7) ]DECYL RADICAL AS A LIGAND IN HYDROFORMYLATION
COMPLEXE METALLIQUE PORTANT UN RESTE 2-PHOSPHA-TRICYCLO 3.3.1.1 3,7 ]DECYLE COMME LIGAND DANS L'HYDROFORMYLATION

(30) Priorität: 12.05.2000 DE 10023468
(43) Veröffentlichungstag der Anmeldung: 05.02.2003
(73) Patentinhaber: BASF AKTIENGESELLSCHAFT, 67056 Ludwigshafen (DE)
(72) Erfinder: AHLERS, Wolfgang, 67549 Worms (DE)
(74) Vertreter: Kinzebach, Werner, Dr.
(86) Internationale Anmeldenummer: PCT/EP2001/005405
(87) Internationale Veröffentlichungsnummer: WO 2001/085661

(56) Entgegenhaltungen:
- WO-A-98/42717
- GB-A- 1 573 422
- GEE, VICTORIA ET AL: "Bis(phospha-adamantyl)alkanes: a new class of very bulky diphosphines" CHEM. COMMUN. (CAMBRIDGE), Nr. 10, 1999, Seiten 901-902, XP001010653 in der Anmeldung erwähnt
- CARRAZ, CHARLES-ANTOINE ET AL: "Titanium Complexes of Sterically Demanding Cage-Phosphinimide Ligands" ORGANOMETALLICS, Bd. 19, Nr. 19, 2000, Seiten 3791-3796, XP002173920

## Beschreibung

Die vorliegende Erfindung betrifft ein verfahren zur Hydroformylierung ethylenisch ungesättigter Verbindungen, bei dem man wenigstens eine ethylenisch ungesättigte Verbindung in Gegenwart eines Ligand-Metall-Komplexes von Ruthenium, Rhodium, Palladium, Iridium und/oder Platin mit Kohlenmonoxid und Wasserstoff umsetzt, sowie einen für das Verfahren geeigneten Ligand-Metall-Komplex.

Die Hydroformylierung oder Oxo-Synthese ist ein wichtiges großtechnisches Verfahren und dient der Herstellung von Aldehyden durch Umsetzung von ethylenisch ungesättigten Verbindungen mit Kohlenmonoxid und Wasserstoff. Die Reaktion selbst ist stark exotherm und läuft im Allgemeinen unter erhöhtem Druck und bei erhöhten Temperaturen in Gegenwart von Katalysatoren ab. In der industriellen Praxis fanden zunächst Cobalt-Katalysatoren Anwendung, in der Zwischenzeit haben sich jedoch überwiegend Rhodium-Katalysatoren etabliert. Um eine Stabilisierung des Rhodium-haltigen Katalysators während der Umsetzung zu bewirken und seine Zersetzung unter Abscheidung von metallischem Rhodium bei der Aufarbeitung zu vermeiden, werden im Allgemeinen Phosphor-haltige Liganden als Cokatalysatoren mit verwendet. Für niedere α-Olefine hat sich insbesondere der Einsatz von Triphenylphosphan und anderen Triarylphosphanen als Cokatalysatoren bewährt (vgl. z. B. J. Falbe, New Synthesis with Carbonmonoxide, Springer, Berlin, 1980, S. 55ff). Obgleich niedere α-Olefine sehr gut mit Triarylphosphan-modifizierten Rhodium-Katalysatoren hydroformyliert werden können, ist dieses Katalysatorsystem für interne und interne verzweigte Olefine sowie für höhere α-Olefine wenig geeignet. So werden interne und interne verzweigte Doppelbindungen nur sehr langsam in Gegenwart eines derartigen Katalysators hydroformyliert. Daher ist bei der Hydroformylierung von Substraten mit internen und/oder internen verzweigten Doppelbindungen die Anwendung hoher Drücke und/oder Temperaturen erforderlich.

Die WO 98/42717 beschreibt Carbonylierungsreaktionen in Gegenwart von Diphosphinen, wovon wenigstens ein Phosphoratom Bestandteil einer 2-Phospha-tricyclo[3.3.1.1{3,7}]decylgruppe ist.

Die FR 118524 beschreibt unter anderem die Hydroformylierung olefinischer Verbindungen in Gegenwart eines Cobaltcarbonyl-Katalysators und eines Cokatalysators in Form eines Trioxaphosphaadamantans.

Der vorliegenden Erfindung liegt die Aufgabe zugrunde, ein Verfahren zur Hydroformylierung ethylenisch ungesättigter Verbindungen unter Katalyse eines Ligand-Metall-Komplexes von Ruthenium, Rhodium, Palladium, Iridium und/oder Platin anzugeben, mit dem ethylenisch ungesättigte Verbindungen, insbesondere ethylenisch ungesättigte Verbindungen mit internen und/oder internen verzweigten Doppelbindungen, unter möglichst wenig stringenten Druck- und/oder Temperaturbedingungen umgesetzt werden können.

Erfindungsgemäß wird diese Aufgabe gelöst durch ein Verfahren zur Hydroformylierung ethylenisch ungesättigter Verbindungen, bei dem man wenigstens eine ethylenisch ungesättigte Verbindung in Gegenwart eines Ligand-Metall-Komplexes von Ruthenium, Rhodium, Palladium, Iridium und/oder Platin mit Kohlenmonoxid und Wasserstoff umsetzt, wobei der Ligand-Metall-Komplex einen Monophosphin-, Monophosphinit- oder Monophosphinamidit-Liganden der allgemeinen Formel I 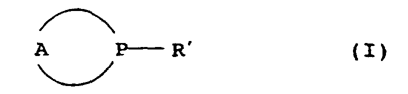 umfasst, worin
- A: zusammen mit dem Phosphoratom, an das es gebunden ist, einen 2-Phospha-tricyclo[3.3.1.1{3,7}]decylrest bildet, in dem gegebenenfalls ein oder mehrere nichtbenachbarte Kohlenstoffatome durch Heteroatome ersetzt sind und der gegebenenfalls substituiert ist, und
- R': für Wasserstoff oder einen über ein Kohlenstoffatom, Sauerstoffatom oder Stickstoffatom gebundenen organischen Rest eines Molekulargewichts von bis zu 20 000 steht, wobei der Rest R' Kein Kein Phosphoratom umfasst und wobei der Ligand die allgemeine Formel II (siehe Seite 4) aufweist.

Die Erfindung betrifft außerdem einen Metall-Ligand-Komplex, der ein unter Ruthenium, Rhodium, Palladium, Iridium und/oder Platin ausgewähltes Metall und wenigstens einen oben definierten Monophosphin- oder Monophosphinit-Liganden umfasst.

Es hat sich gezeigt, dass ethylenisch ungesättigte Verbindungen, insbesondere solche mit internen verzweigten Doppelbindungen, mit dem erfindungsgemäßen Verfahren bei deutlich niedrigeren Temperaturen und/oder Drücken umgesetzt werden können, als zur Hydroformylierung der gleichen Substrate unter Verwendung des gleichen katalytisch aktiven Metalls mit anderen phosphorhaltigen Cokatalysatoren, wie Triarylphosphinen, erforderlich sind. Insbesondere ist mit dem erfindungsgemäßen Verfahren die Hydroformylierung ethylenisch ungesättigter Verbindungen, insbesondere solcher mit internen verzweigten Doppelbindungen, bei Drücken von weniger als 100 bar, vorzugsweise weniger als 80 bar, möglich.

Tricyclo[3.3.1.1{3,7}]decan ist auch unter dem Trivialnamen "Adamantan" bekannt. In dem 2-Phospha-tricyclo[3.3.1.1{3,7}]decylrest des erfindungsgemäß verwendeten Liganden können ein oder mehrere nichtbenachbarte Kohlenstoffatome, die vorzugsweise nicht in Nachbarstellung zum Phosphoratom stehen, durch Heteroatome, vorzugsweise Sauerstoffatome und/oder Stickstoffatome, ersetzt sein. Vorzugsweise sind die Kohlenstoffatome in den Positionen 6, 9 und 10 durch Heteroatome, insbesondere Sauerstoffatome, ersetzt.

Der 2-Phospha-tricyclo[3.3.1.1{3,7}]decylrest kann mit z. B. 1 bis 8, vorzugsweise 1 bis 4, Substituenten substituiert sein. Er kann insbesondere an einem oder mehreren seiner Kohlenstoffatome Substituenten tragen. Vorzugsweise tragen ein oder mehrere Kohlenstoffatome an den Positionen 1, 3, 5 und/oder 7 , insbesondere alle Kohlenstoffatome an den Positionen 1, 3, 5 und 7 Substituenten, die vorzugsweise identisch sind. Geeignete Substituenten sind z. B. Halogen, Alkyl, Cycloalkyl, Halogenalkyl, Aryl und oder Aralkyl. Die Kohlenstoffatome an den Positionen 4 und/oder 8 können einen oder zwei Substituenten, z. B. C₁-C₄-Alkyl oder Halogenatome, insbesondere Fluoratome, tragen.

Der Rest R' steht für Wasserstoff oder einen über ein Kohlenstoffatom, sauerstoffatom oder Stickstoffatom gebundenen organischen Rest eines Molekulargewichts von bis zu 20 000, vorzugsweise bis zu 10 000, insbesondere bis zu 5 000. Bei den erfindungsgemäß eingesetzten Liganden handelt es sich um Monophosphine, Monophosphinite oder Monophosphinamidite, d. h. der Rest R' umfasst kein Phosphoratom. Als Reste R' kommen insbesondere Alkyl, z. B. Alkyl mit 1 bis 500 Kohlenstoffatomen, das durch nicht benachbarte Heteroatome, insbesondere Sauerstoffatome und/oder Stickstoffatome unterbrochen sein kann; Cycloalkyl, Aryl, Aralkyl, Alkoxy, Cycloalkoxy, Aryloxy, Aralkyloxy oder Acyl in Betracht. Außerdem kann R' für eine polymere Kette, z. B. Z(CHR"CH₂O)ₓR"'oder Z(CH₂CH₂NR")ₓR'" stehen, wobei Z für ein Brückenglied aus 0 bis 20 Atomen steht, wovon benachbarte Atome Bestandteil eines gesättigten oder ungesättigten, carbocyclischen oder heterocyclischen Rings sein können steht und R" und R'" unabhängig voneinander für Wasserstoff, Alkyl, Cycloalkyl, Aryl, Aralkyl oder Acyl stehen und x für eine ganze Zahl von 1 bis 240, vorzugsweise 1 bis 60, steht. Beispiele für Brückenglieder Z sind -CH₂-CH₂-O-, -CH₂-CH₂-N(R")-, -CH₂-CH₂- und dergleichen.

Die Liganden weisen die allgemeine Formel II auf 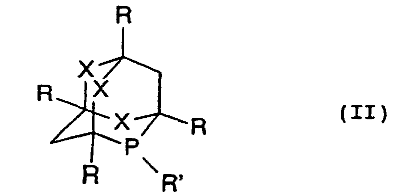 worin die Reste
- X: unabhängig voneinander für O oder NR stehen,
- R: unabhängig voneinander für Wasserstoff, Alkyl, Cycloalkyl, Halogenalkyl, Aryl oder Aralkyl stehen und
- R': für Wasserstoff, Alkyl, Cycloalkyl, Aryl, Aralkyl, Alkoxy, Cycloalkoxy, Aryloxy, Aralkyloxy, Alkylamino, Di(alkyl)amino, Cycloalkylamino, N-Cycloalkyl-N-alkylamino, Di(cycloalkyl)amino, Arylamino, N-Aryl-N-alkylamino, Di(aryl)amino, Aralkylamino, N-Aralkyl-N-alkylamino, N-Aralkyl-N-arylamino, Di(aralkyl)amino, Acyl oder Carbamoyl steht.

Im Rahmen der vorliegenden Anmeldung bedeuten, soweit nicht anders angegeben, die Ausdrücke (auch in Wortzusammensetzungen, wie Alkylamino und dergleichen):
"Alkyl" geradkettiges oder verzweigtes Alkyl, vorzugsweise C₁-C₂₀-Alkyl, insbesondere C₁-C₈-Alkyl, besonders bevorzugt C₁-C₄-Alkyl. Beispiele für Alkylgruppen sind insbesondere Methyl, Ethyl, Propyl, Isopropyl, n-Butyl, 2-Butyl, s-Butyl, t-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl, n-Heptyl, 2-Heptyl, 3-Heptyl, 2-Ethylpentyl, 1-Propylbutyl, Octyl;
"Cycloalkyl" vorzugsweise C₅-C₇-Cycloalkyl, wie Cyclopentyl, Cyclohexyl oder Cycloheptyl;
"Halogenalkyl" vorzugsweise C₁-C₄-Halogenalkyl, d. h. einen C₁-C₄-Alkylrest, der partiell oder vollständig durch Fluor, Chlor, Brom und/oder Iod substituiert ist, wie Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2,2-Difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
"Aryl" vorzugsweise C₆-C₁₆-Aryl, wie Phenyl, Tolyl, Xylyl, Mesityl, Naphthyl, Anthracinyl, Phenantrenyl, Naphthacenyl; insbesondere Phenyl oder Naphthyl;
"Aralkyl" vorzugsweise C₇-C₂₀-Aralkyl, insbesondere Phenyl-C₁-C₄-alkyl, wie Benzyl oder Phenethyl;
"Alkoxy" vorzugsweise C₁-C₂₀-Alkoxy mit einer Alkylgruppe vorzugsweise wie vorstehend definiert;
"Cycloalkyloxy" vorzugsweise C₅-C₇-Cycloalkyloxy mit einer Cycloalkylgruppe vorzugsweise wie vorstehend definiert;
"Aryloxy" vorzugsweise C₇-C₁₆-Aryloxy mit einer Arylgruppe vorzugsweise wie vorstehend definiert;
"Aralkyloxy" vorzugsweise C₇-C₂₀-Aralkyloxy mit einer Aralkylgruppe vorzugsweise wie vorstehend definiert; und
"Acyl" vorzugsweise C₁-C₂₁-Acyl, wie Formyl oder C₁-C₂₀-Alkylcarbonyl mit einer Alkylgruppe vorzugsweise wie vorstehend definiert.
"Carbamoyl" vorzugsweise C₁-C₂₁-Carbamoyl, wie Aminocarbonyl, C₁-C₂₀-Alkylaminocarbonyl, C₆-C₁₆-Arylaminocarbonyl oder C₇-C₂₀-Aralkylaminocarbonyl mit einer Alkyl-, Aryl- oder Aralkylgruppe vorzugsweise wie vorstehend definiert.

Die Reste X stehen vorzugsweise für O, NCH₃ oder NH, insbesondere für O.

Besonders bevorzugt stehen die Reste R unabhängig voneinander für C₁-C₄-Alkyl, C₁-C₄-Halogenalkyl oder Phenyl, insbesondere Methyl, t-Butyl, Trifluormethyl oder Phenyl.

Besonders bevorzugte Liganden sind unter 2-Cyclohexyl-2-phospha-1,3,5,7-tetramethyl-6,9,10-trioxa-tricyclo[3.3.1.1{3,7}]decan, 2-Phenyl-2-phospha-1,3,5,7-tetramethyl-6,9,10-trioxa-tricyclo[3.3.1.1{3,7}]decan, 2-t-Butyl-2-phospha-1,3,5,7-tetramethyl-6,9,10-trioxa-tricyclo[3.3.1.1{3,7}]decan, 2-Octyl-2-phospha-1,3,5,7-tetramethyl-6,9,10-trioxa-tricyclo[3.3.1.1{3,7}]-decan und 2-Phospha-1,3,5,7-tetramethyl-6,9,10-trioxa-tricyclo[3.3.1.1{3,7}]decan ausgewählt.

Zur Herstellung 6,9,10-trioxasubstituierter Liganden der Formel I kann man z. B. Phosphan oder ein primäres Phosphin mit einem 1,3-Diketon, z. B. 2,4-Pentandion oder substituierten 2,4-Pentandionen, wie Perfluor-2,4-pentandion oder 1,1,1,5,5,5-Hexafluor-2,4-pentandion, unter Säurekatalyse umsetzen. Die Verbindungen der Formel I werden im Allgemeinen in hoher Reinheit erhalten und können ohne weitere Aufreinigung unmittelbar verwendet werden. Bezüglich geeigneter Reaktionsbedindungen wird auf J. Am. Chem. Soc. 1961, Vol. 83, 3279-3282 und Chem. Comm. 1999 (10), 901-902 verwiesen.

Im Allgemeinen werden unter Hydroformylierungsbedingungen aus den eingesetzten Katalysatoren oder Katalysatorvorstufen katalytisch aktive Species der allgemeinen Formel HₓM_{y}(CO)_{z}L_{q} gebildet, worin M für Ruthenium, Rhodium, Palladium, Iridium und/oder Platin, L für einen Liganden der allgemeinen Formel I und q, x, y, z für ganze Zahlen in Abhängigkeit von der Wertigkeit und Art des Metalls stehen. Die Komplexe können gewünschtenfalls zusätzlich weitere Liganden aufweisen, die vorzugsweise ausgewählt sind unter Halogeniden, Aminen, Carboxylaten, Acetylacetonat, Aryl- und Alkylsulfonaten, Olefinen, Dienen, Cycloolefinen, Nitrilen, stickstoffhaltigen Heterocyclen, Aromaten und Heteroaromaten, Ether, PF₃ sowie ein-, zwei- und mehrzähnigen Phosphin-, Phosphinit-, Phosphonit- und Phosphit-Liganden, die nicht der Formel I entsprechen.

Nach einer bevorzugten Ausführungsform werden die Hydroformylierungskatalysatoren in situ in dem für die Hydroformylierungsreaktion eingesetzten Reaktor hergestellt. Gewünschtenfalls können die Ligand-Metall-Komplexe jedoch auch separat hergestellt und nach üblichen Verfahren isoliert werden. Zur in situ-Herstellung kann man z. B. wenigstens einen Ligand der Formel I, eine Verbindung unter einem Komplex von Ruthenium, Rhodium, Palladium, Iridium und/oder Platin, gegebenenfalls wenigstens einen weiteren Liganden und gegebenenfalls ein Aktivierungsmittel in einem inerten Lösungsmittel mit Kohlenmonoxid und Wasserstoff unter Hydroformylierungsbedingungen umsetzen.

Geeignete Rhodiumverbindungen oder -komplexe sind z. B. Rhodium(II)- und Rhodium(III)-Salze, wie Rhodium(III)-chlorid, Rhodium(III)-nitrat, Rhodium(III)-sulfat, Kaliumrhodiumsulfat, Rhodium(II)- bzw. Rhodium(III)-carboxylate, wie Rhodium(II)- und Rhodium(III)-acetat, Rhodium(III)-oxid, Salze der Rhodium(III)-säure, Trisammoniumhexachlororhodat(III) usw. Weiterhin eignen sich Rhodiumkomplexe, wie Rhodiumbiscarbonylacetylacetonat, Acetylacetonatobisethylenrhodium(I) usw. Vorzugsweise werden Rhodiumbiscarbonylacetylacetonat oder Rhodiumacetat eingesetzt.

Ebenfalls geeignet sind Rutheniumsalze oder -verbindungen. Geeignete Rutheniumsalze sind beispielsweise Ruthenium(III)-chlorid, Ruthenium(IV)-, Ruthenium(VI)- oder Ruthenium(VIII)-oxid, Alkalisalze der Rutheniumsauerstoffsäuren, wie K₂RuO₄ oder KRuO₄ oder Komplexverbindungen, wie RuHCl(CO)(PPh₃)₃. Auch können die Metallcarbonyle des Rutheniums, wie Trisrutheniumdodecacarbonyl oder Hexarutheniumoctadecacarbonyl oder Mischformen, in denen CO teilweise durch Triorganophosphine ersetzt sind, wie Ru(CO)(PPh₃)₂ verwendet werden.

Geeignete Palladium-, Iridium- und Platin-Verbindungen und -komplexe sind dem Fachmann bekannt und in der Literatur hinreichend beschrieben. Vorzugsweise handelt es sich bei dem Metall um Rhodium.

Geeignete Aktivierungsmittel sind z. B. Brönsted-Säuren, LewisSäuren, wie BF₃, AlCl₃, ZnCl₂ und Lewis-Basen.

Das Molmengenverhältnis von Ligand der Formel I zu Metall liegt im Allgemeinen in einem Bereich von etwa 50:1 bis 1:1, vorzugsweise 10:1 bis 1:1.

Als Substrate für das erfindungsgemäße Verfahren kommen prinzipiell alle Verbindungen in Betracht, welche eine oder mehrere ethylenisch ungesättigte Doppelbindungen enthalten. Dazu zählen z. B. Olefine, wie α-Olefine, interne lineare und interne verzweigte Olefine. Geeignete α-Olefine sind z. B. Ethylen, Propen, 1-Buten, 1-Penten, 1-Hexen, 1-Hepten, 1-Octen, 1-Nonen, 1-Decen, 1-Undecen, 1-Dodecen usw.

Geeignete interne lineare Olefine sind vorzugsweise C₄-C₂₀-Olefine, wie 2-Buten, 2-Penten, 2-Hexen, 3-Hexen, 2-Hepten, 3-Hepten, 2-Octen, 3-Octen, 4-Octen usw.

Geeignete interne verzweigte Olefine sind vorzugsweise C₅-C₂₀-Olefine, wie 2-Methyl-2-buten, 2-Methyl-2-penten, 3-Methyl-2-penten.

Bevorzugte Einsatzmaterialien sind Oligo- oder Poly(C₃-C₆-alkene), bei denen es sich um die Oligomerisations- oder Polymerisationsprodukte von C₃-C₆-Alkenen, wie Propen, 1-Buten, 2-Buten, Isobuten, Penten oder Hexenen, handelt. Bevorzugte Beispiele sind Trimerpropen, Dimerbuten, Trimerbuten und Dimerhexen sowie Polyisobuten mit 20 bis 400 Kohlenstoffatomen. Bei den Oligo- oder Poly(C₃-C₆-alkenen) handelt es sich in der Regel um Gemische im Wesentlichen einfach ungesättigter Olefinisomere mit einem Anteil interner verzweigter Olefine.

Geeignete zu hydroformylierende Olefine sind weiterhin Vinylaromaten, wie Styrol, α-Methylstyrol, 4-Isobutylstyrol usw.

Geeignete ethylenisch ungesättigte Verbindungen sind weiterhin α,β-ethylenisch ungesättigte Mono- und Dicarbonsäuren, deren Ester, Halbester und Amide, Diacrylsäure, Methacrylsäure, Maleinsäure, Fumarsäure, Crotonsäure, Itaconsäure, 3-Pentensäuremethylester, 4-Pentensäuremethylester, Ölsäuremethylester, Acrylsäurealkylester, Methacrylsäurealkylester, ungesättigte Nitrile, wie 3-Pentennitril, 4-Pentennitril, Acrylnitril, vinylether, wie Vinylmethylether, Vinylethylether, Vinylpropylether usw.

Die Vorteile des erfindungsgemäßen Verfahrens kommen besonders zum Tragen, wenn man als ethylenisch ungesättigte Verbindung wenigstens eine lineare Verbindung mit interner Doppelbindung, d. h. eine Verbindung der allgemeinen Formel III, und/oder wenigstens eine Verbindung mit interner verzweigter Doppelbindung, d. h. eine Verbindung der allgemeinen Formel IV, verwendet. 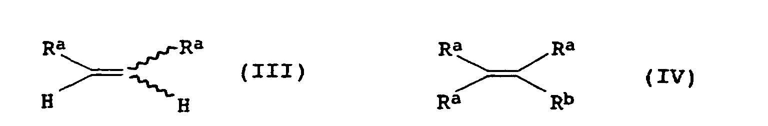 worin die Reste
- R^{a}: unabhängig voneinander für von Wasserstoff verschiedene Reste, insbesondere Alkyl, stehen und
- R^{b}: für Wasserstoff oder einen von Wasserstoff verschiedenen Rest, insbesondere Alkyl, steht.

Die Summe der Kohlenstoffzahlen der Reste R^{a} in der Formel III beträgt vorzugsweise 2 bis 30, insbesondere 2 bis 12. Die Summe der Kohlenstoffzahlen der Reste R^{a} und R^{b} in der Formel IV beträgt vorzugsweise 3 bis 30, insbesondere 3 bis 12. Bevorzugte Beispiele derartiger Verbindungen sind die oben angesprochenen internen linearen und internen verzweigten Olefine.

Die Hydroformylierungsreaktion kann kontinuierlich, semikontinuierlich oder diskontinuierlich erfolgen. Geeignete Reaktoren sind dem Fachmann bekannt und werden z. B. in Ullmanns Enzyklopädie der Technischen Chemie, Band 1, 3. Auflage, 1951, S. 743ff beschrieben.

Kohlenmonoxid und Wasserstoff werden üblicherweise in Form eines Gemisches, dem sogenannten Synthesegas eingesetzt. Das molare Verhältnis von Kohlenmonoxid und Wasserstoff beträgt in der Regel etwa 5:95 bis 70:30, bevorzugt etwa 40:60 bis 60:40. Insbesondere wird ein molares Verhältnis von Kohlenmonoxid und Wasserstoff im Bereich von etwa 1:1 eingesetzt.

Die Temperatur bei der Hydroformylierungsreaktion liegt im Allgemeinen in einem Bereich von etwa 80 bis 180 °C, vorzugsweise etwa 100 bis 160 °C. Die Reaktion wird in der Regel bei dem Partialdruck des Reaktionsgases bei der gewählten Reaktionstemperatur durchgeführt. Im Allgemeinen liegt der Druck in einem Bereich von etwa 10 bis 100 bar, insbesondere 10 bis 80 bar. Die optimale Temperatur und der optimale Druck sind von der eingesetzten ethylenisch ungesättigten Verbindung abhängig. So werden α-Olefine besonders bevorzugt bei Temperaturen von 80 bis 120 °C und einem Druck von 10 bis 40 bar hydroformyliert. Interne und interne verzweigte Olefine werden bevorzugt bei Temperaturen von 120 bis 180 °C und einem Druck von 2 bis 80 bar hydroformyliert, wobei interne lineare Olefine insbesondere bei 2 bis 20 bar und interne verzweigte Olefine insbesondere bei 40 bis 80 bar umgesetzt werden.

Die katalytisch aktiven Ligand-Metall-Komplexe lassen sich nach üblichen, dem Fachmann bekannten Verfahren vom Austrag der Hydroformylierungsreaktion abtrennen und können im Allgemeinen, gegebenenfalls nach Aufarbeitung, erneut für die Hydroformylierung eingesetzt werden.

Bei der Hydroformylierung können Lösungsmittel mitverwendet werden, wie die hochsiedenden Folgereaktionsprodukte der Aldehyde, die bei der Hydroformylierung entstehen. Ebenfalls geeignete Lösungsmittel sind aromatische Kohlenwasserstoffe, wie Toluol und Xylol, aliphatische Kohlenwasserstoffe, Ether, wie 2,5,8-Trioxanonan (Diglyme), Diethylether und Anisol, Sulfone, wie Sulfolan, oder Ester, wie 3-Hydroxy-2,2,4-trimethylpentyl-1-isobutyrat (Texanol).

Die Erfindung wird durch die folgenden, nicht einschränkenden Beispiele näher veranschaulicht:

### Beispiele

Die Synthese der Liganden erfolgte analog zu der in J. Am. Chem. Soc. 1961, Vol. 83, 3279 + 3282 und Chem. Colum. 1999 (10) 901-902 beschriebenen Herstellungsweise. Das in den Beispielen verwendete Dimerbuten wurde durch Dimerisierung eines 1-Buten und 2-Buten enthaltenden C₄-Kohlenwasserstoffgemisches an einem heterogenen, Nickel enthaltenden Katalysator erhalten. Es enthielt etwa 20 Gew.-% interne verzweigte Olefine. Die Abkürzung "acac" steht für Acetylacetonat; "L:M" steht für das molare Verhältnis von Ligand zu Metall.

### Beispiel 1:

### Hydroformylierung von 1-Octen mit 2-Cyclohexyl-2-phospha-1,3,5,7-tetramethyl-6,9,10-trioxa-tricyclo[3.3.1.1{3,7}]decan

0,9 mg Rh(CO)₂acac und 52 mg 2-Cyclohexyl-2-phospha-1,3,5,7-tetramethyl-6,9,10-trioxa-tricyclo[3.3.1.1{3,7}]decan (60 ppm Rh, L:M = 50:1) wurden zusammen eingewogen, in 3 g Toluol gelöst und bei 100 °C mit 10 bar Synthesegas (CO:H₂ = 1:1) begast. Nach 30 min. wurde entspannt, dann wurden 3 g 1-Octen zugegeben und 4 h bei 100 °C und 10 bar hydroformyliert. Der Umsatz betrug 98 %, die Aldehydselektivität 99 %.

### Beispiel 2:

### Hydroformylierung von Dimerbuten mit 2-Cyclohexyl-2-phospha-1,3,5,7-tetramethyl-6,9,10-trioxa-tricyclo[3.3.1.1{3,7}]decan

3 mg Rh(CO)₂acac und 35 mg 2-Cyclohexyl-2-phospha-1,3,5,7-tetramethyl-6,9,10-trioxa-tricyclo[3.3.1.1{3,7}]decan (60 ppm Rh, L:M = 10:1) wurden zusammen eingewogen, in 10 g Toluol gelöst und bei 100 °C mit 10 bar Synthesegas (CO:H₂ = 1:1) begast. Nach 30 min. wurde entspannt, dann wurden 10 g Dimerbuten zugegeben und 4 h bei 160 °C und 60 bar hydroformyliert. Der Umsatz betrug 94 %, die Nonanalselektivität 99 % und die Nonanolselektivität 1 %.

### Beispiel 3:

### Hydroformylierung von Dimerbuten mit 2-Cyclohexyl-2-phospha-1,3,5,7-tetramethyl-6,9,10-trioxa-tricyclo[3.3.1.1{3,7}] decan

3 mg Rh(CO)₂acac und 35 mg 2-Cyclohexyl-2-phospha-1,3,5,7-tetramethyl-6,9,10-trioxa-tricyclo[3.3.1.1{3,7}]decan (60 ppm Rh, L:M = 10:1) wurden zusammen eingewogen, in 10 g Toluol gelöst und bei 100 °C mit 10 bar Synthesegas (CO:H₂ = 1:1) begast. Nach 30 min. wurde entspannt, dann wurden 10 g Dimerbuten zugegeben und 4 h bei 140 °C und 60 bar hydroformyliert. Der Umsatz betrug 94 %, die Nonanalselektivität 99 %, die Nonanolselektivität 1 %.

### Beispiel 4:

### Hydroformylierung von 1-Octen mit 2-Phospha-1,3,5,7-tetramethyl-6,9,10-trioxa-tricyclo[3.3.1.1{3,7}]decan

3 mg Rh(CO)₂acac und 125,6 mg 2-Phospha-1,3,5,7-tetramethyl-6,9,10-trioxa-tricyclo[3.3.1.1{3,7}]decan (60 ppm Rh, L:M = 50:1) wurden separat, in insgesamt 10 g Toluol gelöst, vermischt und bei 100 °C mit 10 bar Synthesegas (CO:H₂ = 1:1) begast. Nach 30 min. wurde entspannt, dann wurden 10 g 1-Octen zugegeben und 4 h bei 100 °C und 10 bar hydroformyliert. Der Umsatz betrug 29 %, die Aldehydselektivität 100 %.

### Beispiel 5:

### Hydroformylierung von Dimerbuten mit 2-Phospha-1,3,5,7-tetramethyl-6,9,10-trioxa-tricyclo[3.3.1.1{3,7}]decan

3 mg Rh(CO)₂acac und 25,1 mg 2-Phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100 °C mit 10 bar Synthesegas (CO:H₂ = 1:1) begast. Nach 30 min. wurde entspannt, dann wurden 10 g Dimerbuten zugegeben und 4 h bei 160 °C und 80 bar hydroformyliert. Der Umsatz betrug 88 %, die Nonanalselektivität 89 %, die Nonanolselektivität 2 %.

### Beispiel 6:

### Hydroformylierung von 1-Octen mit 2-Octyl-2-phospha-1,3,5,7-tetramethyl-6,9,10-trioxa-tricyclo[3.3.1.1{3,7}]decan

0,75 mg Rh(CO)₂acac und 28,6 mg 2-Octyl-2-phospha-1,3,5,7-tetramethyl-6,9,10-trioxa-tricyclo[3.3.1.1{3,7}]decan (60 ppm, L:M = 30:1) wurden separat in insgesamt 2,5 g Toluol gelöst, vermischt und bei 100°C mit 10 bar Synthesegas (CO:H₂ = 1:1) begast. Nach 30 min. wurde entspannt, dann wurden 2,5 g 1-Octen zugegeben und 4 h bei 100°C und 10 bar hydroformyliert. Der Umsatz betrug 98%, die Aldehydselektivität 100% und die Linearität 56%.

### Beispiel 7

### Hydroformylierung von Dimerbuten mit 2-Cyclohexyl-2-phospha-1,3,5,7-tetramethyl-6,9,10-trioxa-tricyclo[3.3.1.1{3,7}]decan

3 mg Rh(CO)₂acac und 6,9 mg 2-Cyclohexyl-2-phospha-1,3,5,7-tetramethyl-6,9,10-trioxa-tricyclo[3.3.1.1{3,7}]decan (60 ppm Rh, L:M = 2:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 140°C und 60 bar hydroformyliert. Der Umsatz betrug 74%, die Nonanalselektivität 100%.

### Beispiel 8

### Hydroformylierung von Dimerbuten mit 2-Octyl-2-phospha-1,3,5,7-tetramethyl-6,9,10-trioxa-tricyclo[3.3.1.1{3,7}]decan

3 mg RH(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 140°C und 40 bar hydroformyliert. Der Umsatz betrug 48%, die Nonanalselektivität 100%.

### Beispiel 9

### Hydroformylierung von Dimerbuten mit 2-Octyl-2-phospha-1,3,5,7-tetramethyl-6,9,10-trioxa-tricyclo[3.3.1.1{3,7}]decan

3 mg Rh(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 140°C und 60 bar hydroformyliert. Der Umsatz betrug 74%, die Nonanalselektivität 99%, die Nonanolselektivität 1%.

### Beispiel 10

### Hydroformylierung von Dimerbuten mit 2-Octyl-2-phospha-1,3,5,7-tetramethyl-6,9,10-trioxa-tricyclo[3.3.1.1{3,7}]decan

3 mg Rh(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 140°C und 80 bar hydroformyliert. Der Umsatz betrug 88%, die Nonanalselektivität 99%, die Nonanolselektivität 1%.

### Beispiel 11

### Hydroformylierung von Dimerbuten mit 2-Octyl-2-phospha-1,3,5,7-tetramethyl-6,9,10-trioxa-tricyclo[3.3.1.1{3,7}]decan

2 mg Rh(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 160°C und 40 bar hydroformyliert. Der Umsatz betrug 44%, die Nonanalselektivität 98%, die Nonanolselektivität 2%.

### Beispiel 12

### Hydroformylierung von Dimerbuten mit 2-Octyl-2-phospha-1,3,5,7-tetramethyl-6,9,10-trioxa-tricyclo[3.3.1.1{3,7}]decan

3 mg Rh(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 160°C und 60 bar hydroformyliert. Der Umsatz betrug 72%, die Nonanalselektivität 94%, die Nonanolselektivität 3%, der Aldolanteil 3.

### Beispiel 13

### Hydroformylierung von Dimerbuten mit 2-Octyl-2-phospha-1,3,5,7-tetramethyl-6,9,10-trioxa-tricyclo[3.3.1.1{3,7}]decan

3 mg Rh(CO)₂acac und 38,1 mg 2-Octyl-2-phospha-1,3,5,7-tetramethyl-6,9,10-trioxa-tricyclo[3.3.1.1{3,7}]decan (60 ppm Rh, L:M = 10:1) wurden separat in insgesamt 10 g Toluol gelöst, vermischt und bei 100°C mit 10 bar Synthesegas (CO:H₂ = 1:1) begast. Nach 30 min wurde entspannt, dann wurden 10 g Dimerbuten zugegeben und 4 h bei 160°C und 80 bar hydroformyliert. Der Umsatz betrug 83%, die Nonanalselektivität 89%, die Nonanolselektivität 5%, der Aldolanteil 6%.

### Vergleichsbeispiel A

### Hydroformylierung von Dimerbuten mit 1,3-P,P'-Di(2-phospha-1,3,5,7-tetramethyl-6,9,10-trioxa-tricyclo[3.3.1.1{3,7}]decyl)-propan

3 mg Rh(CO)₂acac und 274 mg 1,3-P,P'-Di(2-phospha-1,3,5,7-tetramethyl-6,9,10-trioxa-tricyclo[3.3.1.1{3,7}]decyl)propan (60 ppm Rh, L:M = 50:1) wurden zusammen eingewogen, in 10 g Toluol gelöst und bei 100 °C mit 10 bar Synthesegas (CO:H₂ = 1:1) begast. Nach 30 min. wurde entspannt, dann wurden 10 g Dimerbuten zugegeben und 4 h bei 160 °C und 80 bar hydroformyliert. Der Umsatz betrug 2 %, die Nonanalselektivität 100 %.

### Vergleichsbeispiel B

### Hydroformylierung von Dimerbuten mit 1,3-P,P'-Di(2-phospha-1,3,5,7-tetramethyl-6,9,10-trioxa-tricyclo[3.3.1.1{3,7}]decyl) propan

Vergleichsbeispiel A wurde mit einem L:M = 10:1 wiederholt. Dabei wurde dasselbe Ergebnis wie in Vergleichsbeispiel A erhalten.

### Vergleichsbeispiel C

### Hydroformylierung von 2-Octen mit 1,3-P,P'-Di(2-phospha-1,3,5,7-tetramethyl-6,9,10-trioxa-tricyclo[3.3.1.1{3,7}]decyl)propan

3 mg Rh(CO)₂acac und 274 mg 1,3-P,P'-Di(2-phospha-1,3,5,7-tetramethyl-6,9,10-trioxa-tricyclo[3.3.1.1{3,7}]decyl)propan (60 ppm Rh, L:M = 50:1) wurden zusammen eingewogen, in 10 g Toluol gelöst und bei 100 °C mit 10 bar Synthesegas (CO:H₂ = 1:1) begast. Nach 30 min. wurde entspannt, dann wurden 10 g 2-Octen zugegeben und 4 h bei 160 °C und 80 bar hydroformyliert. Der Umsatz betrug 14 %, die Aldehydselektivität 79 %.

### Vergleichsbeispiel D

### Hydroformylierung von Dimerbuten mit Triphenylphosphan

7,5 g Rh(CO)₂acac und 157 mg Triphenylphosphan (60 ppm Rh, L:M = 20:1) wurden separat in insgesamt 25 g Toluol gelöst, vermischt und bei 100°C mit 10 bar Synthesegas (CO:H₂ = 1:1) begast. Nach 30 min wurde entspannt, dann wurden 25,1 g Dimerbuten zugegeben und 4 h bei 160°C und 60 bar hydroformyliert. Der Umsatz betrug 9%, die Nonanalselektivität 47%.

## Patentansprüche

1. Verfahren zur Hydroformylierung ethylenisch ungesättigter Verbindungen, bei dem man wenigstens eine ethylenisch ungesättigte Verbindung in Gegenwart eines Ligand-Metall-Komplexes von Ruthenium, Rhodium, Palladium, Iridium und/oder Platin mit Kohlenmonoxid und Wasserstoff umsetzt, wobei der Ligand-Metall-Komplex einen Monophosphin-, Monophosphinit- oder Monophosphinamidit-Liganden der allgemeinen Formel I 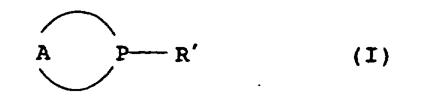 umfasst, worin
A zusammen mit dem Phosphoratom, an das es gebunden ist, einen 2-Phospha-tricyclo[3.3.1.1{3,7}]decylrest bildet, in dem gegebenenfalls ein oder mehrere nichtbenachbarte Kohlenstoffatome durch Heteroatome ersetzt sind und der gegebenenfalls substituiert ist, und
R' für Wasserstoff oder einen über ein Kohlenstoffatom oder Sauerstoffatom oder Stickstoffatom gebundenen organischen Rest eines Molekulargewichts von bis zu 20 000 steht, wobei der Rest R' kein Phosphoratom umfasst*,* und
wobei der Ligand die allgemeine Formel II aufweist 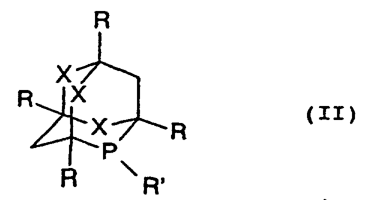 worin die Reste
X unabhängig voneinander für O oder NR stehen,
R unabhängig voneinander für Wasserstoff, Alkyl, Cycloalkyl, Halogenalkyl, Aryl oder Aralkyl stehen und
R' für Wasserstoff, Alkyl, Cycloalkyl, Aryl, Aralkyl, Alkoxy, Cycloalkoxy, Aryloxy, Aralkyloxy, Alkylamino, Di(alkyl)amino, Cycloalkylamino, N-Cycloalkyl-N-alkylamino, Di(cycloalkyl)amino, Arylamino, N-Aryl-N-alkylamino, Di(aryl)amino, Aralkylamino, N-Aralkyl-N-alkylamino, N-Aralkyl-N-arylamino, Di(aralkyl)amino, Acyl oder Carbamoyl steht.

2. Verfahren nach Anspruch 1, wobei die Reste R unabhängig voneinander für Methyl, t-Butyl, Trifluormethyl oder Phenyl stehen.

3. verfahren nach Anspruch 2, wobei der Ligand unter 2-Cyclohexyl-2-phospha-1,3,5,7-tetramethyl-6,9,10-trioxatricyclo[3.3.1.1{3,7}]decan,
2-Octyl-2-phospha-1,3,5,7-tetramethyl-6,9.10-trioxa-tricyclo[3.3.1.1{3,7}]decan, oder
2-Phenyl-2-phospha-1,3,5,7-tetramethyl-6,9,10-trioxa-tricyclo[3.3.1.1{3,7}]decan,
2-t-Butyl-2-phospha-1,3,5,7-tetramethyl-6,9,10-trioxa-tricyclo[3.3.1.1{3,7}]decan oder
2-Phospha-1,3,5,7-tetramethyl-6,9,10-trioxa-tricyclo[3.3.1.1 {3,7}]decan ausgewählt ist.

4. Verfahren nach einem der vorhergehenden Ansprüche, wobei man als ethylenisch ungesättigte Verbindung wenigstens eine Verbindung der allgemeinen Formel III oder IV verwendet, 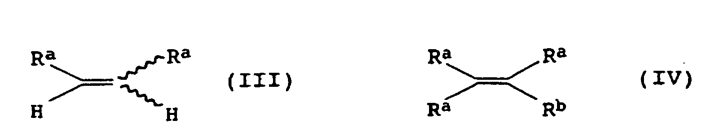 worin die Reste
R^{a} unabhängig voneinander für von Wasserstoff verschiedene Reste stehen und
R^{b} für Wasserstoff oder einen von Wasserstoff verschiedenen Rest steht.

5. Verfahren nach Anspruch 4, wobei man als ethylenisch ungesättigte Verbindung ein Oligo- oder Poly(C₃-C₆-alken) verwendet.

6. Ligand-Metall-Komplex, umfassend ein unter Ruthenium, Rhodium, Palladium, Iridium und/oder Platin ausgewähltes Metall und wenigstens einen Monophosphin-, Monophosphinit- oder Monophosphinamidit-Liganden der allgemeinen Formel I 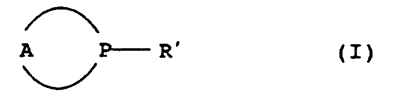 umfasst, worin
A zusammen mit dem Phosphoratom, an das es gebunden ist, ein 2-Phospha-tricyclo[3.3.1.1{3,7}]decylrest bildet, in dem gegebenenfalls ein oder mehrere nichtbenachbarte Kohlenstoffatome durch Heteroatome ersetzt sind und der gegebenenfalls substituiert ist, und
R' für einen über ein Kohlenstoffatom, Sauerstoffatom oder Stickstoffatom gebundenen organischen Rest eines Molekulargewichts von bis zu 20 000 steht, wobei der Rest R' kein Phosphoratom umfasst, und
wobei der Ligand die allgemeine Formel II aufweist 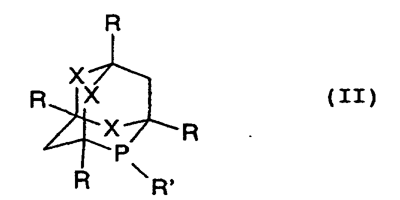 worin die Reste
X unabhängig voneinander für O oder NR stehen,
R unabhängig voneinander für Wasserstoff, Alkyl, Cycloalkyl, Halogenalkyl, Aryl oder Aralkyl stehen und
R' für Wasserstoff, Alkyl, Cycloalkyl, Aryl, Aralkyl, Alkoxy, Cycloalkoxy, Aryloxy, Aralkyloxy, Alkylamino, Di(alkyl)amino, Cycloalkylamino, N-Cycloalkyl-N-alkylamino, Di(cycloalkyl)amino, Arylamino, N-Aryl-N-alkylamino, Di(aryl)amino, Aralkylamino, N-Aralkyl-N-alkylamino, N-Aralkyl-N-arylamino, Di(aralkyl)amino, Acyl oder Carbamoyl steht.

## Claims

1. A process for the hydroformylation of ethylenically unsaturated compounds, in which at least one ethylenically unsaturated compound is reacted with carbon monoxide and hydrogen in the presence of a ligand-metal complex of ruthenium, rhodium, palladium, iridium and/or platinum, wherein the ligand-metal complex comprises a monophosphine, monophosphinite or monophosphinamidite ligand of the formula I 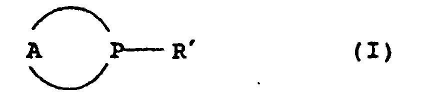 where
A together with the phosphorus atom to which it is bound forms a 2-phosphatricyclo[3.3.1.1{3,7}]decyl radical in which one or more nonadjacent carbon atoms may be replaced by heteroatoms and which may be substituted, and
R' is hydrogen or an organic radical having a molecular weight of up to 20 000 bound via a carbon atom or oxygen atom or nitrogen atom, where the radical R' does not include a phosphorus atom, and
the ligand has the formula II 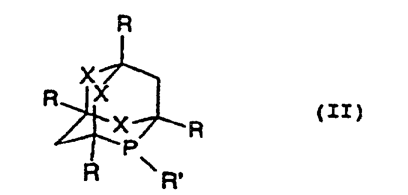
where
X are each, independently of one another, O or NR,
R are each, independently of one another, hydrogen, alkyl, cycloalkyl, haloalkyl, aryl or aralkyl and
R' is hydrogen, alkyl, cycloalkyl, aryl, aralkyl, alkoxy, cycloalkoxy, aryloxy, aralkyloxy, alkylamino, di(alkyl)amino, cycloalkylamino, N-cycloalkyl-N-alkylamino, di(cycloalkyl)amino, arylamino, N-aryl-N-alkylamino, di(aryl)amino, aralkylamino, N-aralkyl-N-alkylamino, N-aralkyl-N-arylamino, di(aralkyl)amino, acyl or carbamoyl.

2. A process as claimed in claim 1, wherein the radicals R are each, independently of one another, methyl, t-butyl, trifluoromethyl or phenyl.

3. A process as claimed in claim 2, wherein the ligand is selected from among 2-cyclohexyl-2-phospha-1,3,5,7-tetramethyl-6,9,10-trioxatricyclo[3.3.1.1{3,7}]decane, 2-octyl-2-phospha-1,3,5,7-tetramethyl-6,9,10-trioxatricyclo
[3.3.1.1{3,7}]decane, 2-phenyl-2-phospha-1,3,5,7-tetramethyl-6,9,10-trioxatricyclo
[3.3.1.1{3,7}]decane, 2-t-butyl-2-phospha-1,3,5,7-tetramethyl-6,9,10-trioxatricyclo[3.3.1.1{3,7}]decane and 2-phospha-2,3,5,7-tetramethyl-6,9,10-trioxatricyclo[3.3.1.1 {3,7}]decane.

4. A process as claimed in any of the preceding claims, wherein at least one compound of the formula III or IV, 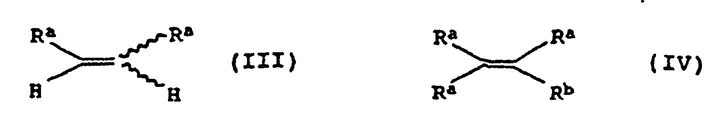 where
R^{a} are, independently of one another, radicals different from hydrogen and
R^{b} is hydrogen or a radical different from hydrogen,
is used as ethylenically unsaturated compound.

5. A process as claimed in claim 4, wherein an oligo(C₃-C₆-alkene) or poly(C₃-C₆-alkene) is used as ethylenically unsaturated compound.

6. A ligand-metal complex comprising a metal selected from among ruthenium, rhodium, palladium, iridium and/or platinum and at least one monophosphine, monophosphinite or monophosphinamidite ligand of the formula I 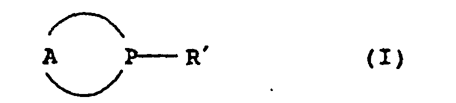 where
A together with the phosphorus atom to which it is bound forms a 2-phosphatricyclo[3.3.1.1{3,7}]decyl radical in which one or more nonadjacent carbon atoms may be replaced by heteroatoms and which may be substituted, and
R' is an organic radical having a molecular weight of up to 20 000 bound via a carbon atom, oxygen atom or nitrogen atom and does not include a phosphorus atom,
wherein the ligand has the formula II 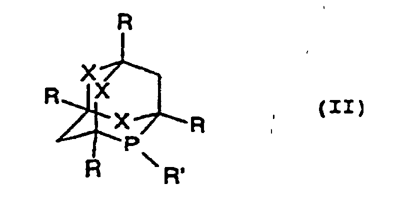 where
X are each, independently of one another, O or NR,
R are each, independently of one another, hydrogen, alkyl, cycloalkyl, haloalkyl, aryl or aralkyl and
R' is hydrogen, alkyl, cycloalkyl, aryl, aralkyl, alkoxy, cycloalkoxy, aryloxy, aralkyloxy, alkylamino, di(alkyl)amino, cycloalkylamino, N-cycloalkyl-N-alkylamino, di(cycloalkyl)amino, arylamino, N-aryl-N-alkylamino, di(aryl)amino, aralkylamino, N-aralkyl-N-alkylamino, N-aralkyl-N-arylamino, di(aralkyl)amino, acyl or carbamoyl.

## Revendications

1. Procédé d'hydroformylation de composés éthyléniquement insaturés, dans lequel au moins un composé éthyléniquement insaturé réagit avec du monoxyde de carbone et de l'hydrogène en présence d'un complexe ligand/métal de ruthénium, rhodium, palladium, iridium et/ou platine, le complexe ligand/métal comportant un ligand de monophosphine, de monophosphinite ou de monophosphinamidite de la formule générale I : 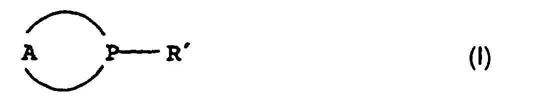 dans laquelle
A forme, avec l'atome de phosphore auquel il est lié, un radical de 2-phospha-tricyclo[3.3.1.1{3,7}]décyle, dans lequel éventuellement un ou plusieurs atomes de carbone non voisins sont remplacés par des hétéroatomes et qui est éventuellement substitué, et
R' représente de l'hydrogène ou un radical organique fixé par l'intermédiaire d'un atome de carbone ou d'un atome d'oxygène ou d'un atome d'azote et présentant un poids moléculaire de jusqu'à 20.000, le radical R' ne comportant pas d'atome de phosphore, et
le ligand présentant la formule générale II : 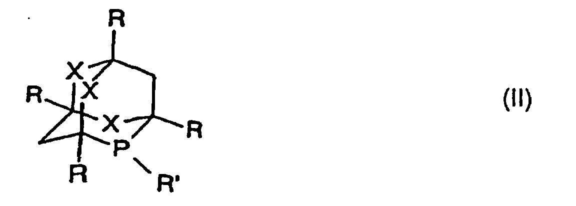
dans laquelle
les radicaux X représentent indépendamment l'un de l'autre O ou NR,
les radicaux R représentent indépendamment l'un de l'autre de l'hydrogène, de l'alkyle, du cycloalkyle, de l'halogénoalkyle, de l'aryle ou de l'aralkyle, et
R' représente de l'hydrogène, de l'alkyle, du cycloalkyle, de l'aryle, de l'aralkyle, de l'alcoxy, du cycloalcoxy, de l'aryloxy, l'aralkyloxy, de l'alkylamino, du di(alkyl)amino, du cycloalkylamino, du N-cycloalkyl-N-alkylamino, du di(cycloalkyl)amino, de l'arylamino, du N-aryl-N-alkylamino, du di(aryl)amino, de l'aralkylamino, du N-aralkyl-N-alkylamino, du N-aralkyl-N-arylamino, du di(aralkyl)amino, de l'acyle ou du carbamoyle.

2. Procédé suivant la revendication 1, dans lequel les radicaux R représentent, indépendamment l'un de l'autre, du méthyle, du t-butyle, du trifluorométhyle ou du phényle.

3. Procédé suivant la revendication 2, dans lequel le ligand est choisi parmi du 2-cyclohexyl-2-phospha-1,3,5,7-tétraméthyl-6,9,10-trioxatricyclo[3.3.1.1{3,7}]décane, du 2-octyl-2-phospha-1,3,5,7-tétraméthyl-6,9,10-trioxatricyclo[3.3.1.1{3,7}]décane, ou du 2-phényl-2-phospha-1,3,5,7-tétraméthyl-6,9,10-trioxatricyclo[3.3.1.1{3,71]décane, du 2-t-butyl-2-phospha-1,3,5,7-tétraméthyl-6,9,10-trioxatricyclo[3.3.1.1{3,7}]décane ou du 2-phospha-1,3,5,7-tétraméthyl-6,9,10-trioxatricyclo[3.3.1.1{3,7}]-décane.

4. Procédé suivant l'une des revendications précédentes, dans lequel, comme composé éthyléniquement insaturé, on utilise au moins un composé de la formule générale III ou IV : 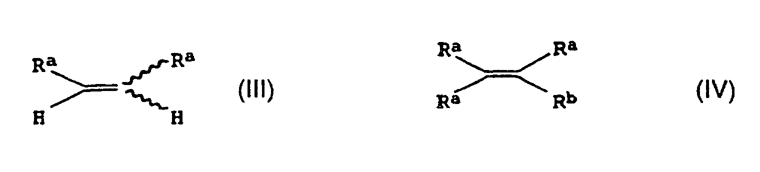 dans lesquelles
les radicaux R^{a} représentent indépendamment l'un de l'autre des radicaux différents de l'hydrogène, et
R^{b} représente de l'hydrogène ou un radical différent de l'hydrogène.

5. Procédé suivant la revendication 4, dans lequel, comme composé éthyléniquement insaturé, on utilise un oligo-(C₃-C₆-alcène) ou un poly-(C₃-C₆-alcène).

6. Complexe ligand-métal, comportant un métal choisi parmi le ruthénium, le rhodium, le palladium, l'iridium et/ou le platine et au moins un ligand de monophosphine, de monophosphinite ou de monophosphinamidite de la formule générale I : 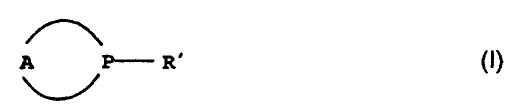 dans laquelle
A forme, avec l'atome de phosphore auquel il est lié, un radical de 2-phospha-tricyclo[3.3.1.1{3,7)]décyle, dans lequel éventuellement un ou plusieurs atomes de carbone non voisins sont remplacés par des hétéroatomes et qui est éventuellement substitué, et
R' représente un radical organique fixé par l'intermédiaire d'un atome de carbone, d'un atome d'oxygène ou d'un atome d'azote et présentant un poids moléculaire de jusqu'à 20.000, le radical R' ne comportant pas d'atome de phosphore,
le ligand présentant la formule générale II : 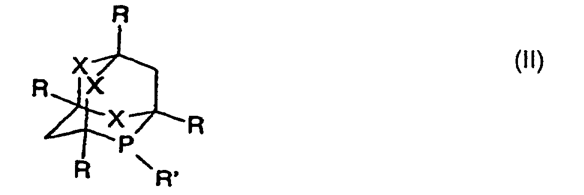 dans laquelle
les radicaux X représentent indépendamment l'un de l'autre O ou NR,
les radicaux R représentent indépendamment l'un de l'autre de l'hydrogène, de l'alkyle, du cycloalkyle, de l'halogénoalkyle, de l'aryle ou de l'aralkyle, et
R' représente de l'hydrogène, de l'alkyle, du cycloalkyle, de l'aryle, de l'aralkyle, de l'alcoxy, du cycloalcoxy, de l'aryloxy, l'aralkyloxy, de l'alkylamino, du di(alkyl)amino, du cycloalkylamino, du N-cycloalkyl-N-alkylamino, du di(cycloalkyl)amino, de l'arylamino, du N-aryl-N-alkylamino, du di(aryl)amino, de l'aralkylamino, du N-aralkyl-N-alkylamino, du N-aralkyl-N-arylamino, du di(aralkyl)amino, de l'acyle ou du carbamoyle.
